# EUROPÄISCHE PATENTSCHRIFT

(11) **EP 2 063 309 B1**
(45) Veröffentlichungstag und Bekanntmachung des Hinweises auf die Patenterteilung: **26.09.2012**
(21) Anmeldenummer: 07022009.0
(22) Anmeldetag: 13.11.2007
(51) Int. Cl.: G02B 27/28, F21S 8/00

(54) **Verfahren zum Ausleuchten einer Operationsstelle**
Method for illuminating an operation point
Procédé d'illumination d'un poste d'opération

(43) Veröffentlichungstag der Anmeldung: 27.05.2009
(73) Patentinhaber: TRUMPF Medizin Systeme GmbH + Co. KG, 07318 Saalfeld (DE)
(72) Erfinder: Rosenheimer, Rouven, 85614 Kirchseeon (DE); Hiemer, Willibald, 81245 München (DE); Schenk, Martin, 72810 Gomaringen (DE)
(74) Vertreter: Hofer, Dorothea

(56) Entgegenhaltungen:
- WO-A-96/17206
- WO-A-2007/014629
- WO-A1-2006/101736
- DE-A1- 3 435 369
- US-A- 2 423 321
- US-A- 4 759 615
- US-A- 6 028 303
- US-A1- 2002 088 927
- US-A1- 2004 227 989
- US-A1- 2006 241 495

## Beschreibung

Die Erfindung betrifft ein Verfahren zum Ausleuchten einer Operationsstelle.

Bei Operationsleuchten steht üblicherweise eine optimale Ausleuchtung des Operationsfelds im Vordergrund. Spezifische Strukturen, wie beispielsweise Lymphgefäße, Tumore, Metastasen, applizierte Stammzellen etc. sind jedoch häufig nicht klar gegenüber dem umgebenden Gewebe abgrenzbar. Sie werden im wesentlichen durch die anatomische Kenntnis und die Erfahrung des Operateurs erkannt. In manchen Fällen sind sie überhaupt nicht makroskopisch erkennbar.

Aus der W02007/014629 A1 ist es bekannt, bei einer Operation eine Unterscheidung von unterschiedlichen Geweben mit Hilfe der Fluoreszenzdiagnostik zu treffen. Eine Operationsleuchte strahlt Licht einer bestimmten Wellenlänge (Referenzlicht) aus, und der rückgestreute Anteil wird zusammen mit dem entstehenden Fluoreszenzlicht detektiert. Die Operationsleuchte kann dazu mit einer elektronischen Kamera kombiniert werden.

Aus der W096/17206 ist es bekannt, einen Polarisationsfilter an einer Operationsleuchte und einen weiteren Polarisationsfilter an der Lupe, dem Augenglas des Operateurs, vorzusehen. Die Polarisationsfilter können drehbar sein.

Aus der DE 680 08 531 ist es bekannt, einen Polarisationsfilter an einer Operationsleuchte zum Verringern des Auftretens störender Reflexe an blanken Instrumenten vorzusehen.

Bei der intraoperativen Fluoreszenzdiagnostik mit einem Kamerasystem entstehen häufig Scheinfluoreszenzen, bei denen es sich um Lichtreflexionen des schmalbandigen Anregungslichts handelt, die die Unterscheidung von reflektierenden Bereichen und fluoreszierenden Bereichen erschweren.

Die Offenlegungsschrift DE 34 35 369 offenbart ein Endoskop mit einer Lichtquelle, einer Kamera, zwei alternativ drehbaren Polarisationsfiltern, einer Auswerteeinheit mit einem Vergleicher, einem Integrator und einem Verstärker. Der Vergleicher vergleicht die von der Kamera abgegebenen Videosignale mit einem vorbestimmten Schwellwert und gibt bei Überschreiten des Schwellwerts ein Ausgangssignal an den Integrator (18) aus, der das Aüsgangssignal für eine Bildperiode integriert, um so den Drehantrieb eines Polarisationsfilters so anzusteuern, dass Überstrahlung vermieden wird.

Es ist Aufgabe der Erfindung, ein Verfahren zum Ausleuchten einer Operationsstelle bereitzustellen, mit dem die Abgrenzung spezifischer Strukturen von dem umliegenden Gewebe an der Operationsstelle während der Operation verbessert wird.

Die Aufgabe wird gelöst durch ein Verfahren nach Patentanspruch 1. Weiterbildungen der Erfindung sind in den abhängigen Ansprüchen angegeben.

Die Erfindung weist den Vorteil auf, daß Zielstrukturen an der Operationsstelle genau und in kürzester Zeit dargestellt werden und gegen das umliegende Gewebe abgegrenzt werden können, ohne daß der Operateur Manipulationen an der Operationsleuchte vornehmen muss. Er kann sich daher noch besser auf die operative Tätigkeit konzentrieren.

Bestehende Operationsleuchten können nachgerüstet werden, so dass ihr Anwendungsgebiet erweitert wird.

Weitere Merkmale und Zweckmäßigkeiten der Erfindung ergeben sich aus der Beschreibung von Ausführungsbeispielen anhand der Figuren. Von den Figuren zeigen:
Fig. 1 eine schematische Darstellung der Operationsleuchte;
Fig. 2a) ein Kamerabild einer Operationsstelle mit fluoreszierenden und reflektierenden Bereichen; und
Fig. 2b) ein Kamerabild der Operationsstelle von Fig. 2a), wobei die Reflexionen eliminiert sind.

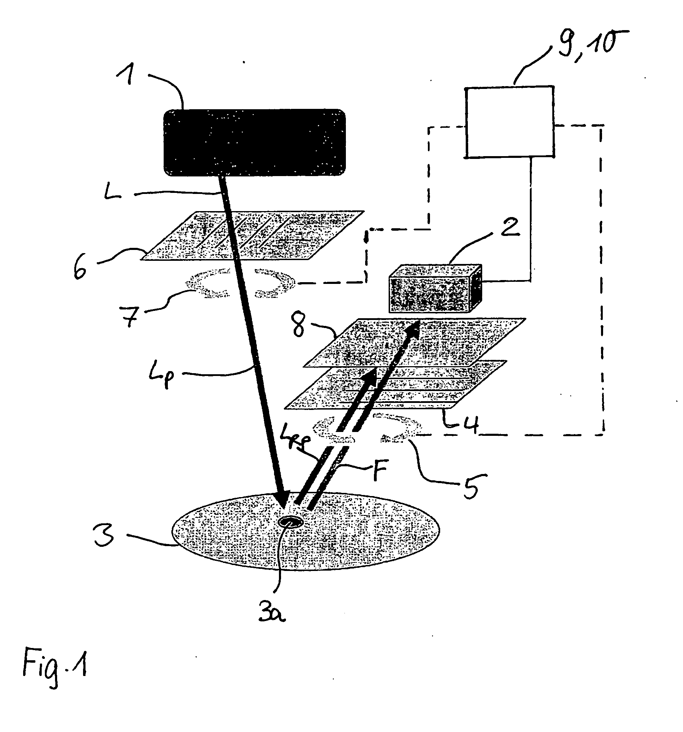

Wie aus Fig. 1 ersichtlich ist, weist die Operationsleuchte eine Lichtquelle 1 und einen Detektor in Form einer Kamera 2 auf. Die Lichtquelle 1 beinhaltet eine Quelle für schmalbandiges Licht bestimmter Wellenlängen zum Anregen eines Fluorophors. Als Lichtquelle werden z.B. LEDs verwendet. Für das Fluorophor Fluorescein wird als Lichtquelle eine blaue LED verwendet, die unpolarisiertes Licht von 450 ± 5 Nanometer ausstrahlt. Der Wellenlängenbereich des von der Lichtquelle ausgesandten Lichts bei Verwendung einer royalblauen LED liegt somit bei 445 - 455 nm. Vorteilhafterweise wird der Wellenlängenbereich des Lichts der Lichtquelle in Entsprechung zu dem Bereich gewählt, in dem der Fluorophor empfindlich ist. Die Lichtquelle kann jede beliebige Form aufweisen. Insbesondere kann eine flächige Beleuchtung durch eine Vielzahl von LEDs realisiert werden. Die Lichtquelle wird üblicherweise oberhalb einer Operationsstelle 3 angeordnet.

Der Detektor 2 ist bevorzugt als CCD-Kamera ausgebildet, die ein Bild der Operationsstelle 3 aufnimmt. Es ist vorteilhaft, wenn die Kamera über eine integrierte Bildausrichtungseinheit und einen Autofokus verfügt.

Vor dem Objektiv der Kamera ist ein erster Polarisationsfilter 4, der den Analysator bildet, angeordnet, der über eine schematisch dargestellte Antriebseinheit 5 drehbar ist. Zwischen der Lichtquelle 1 und der Operationsstelle 3 ist ferner ein zweiter Polarisationsfilter 6, der den Polarisator bildet, vorgesehen, der über eine schematisch dargestellte Antriebseinheit 7 drehbar ist.

Ferner ist bei dem gezeigten Ausführungsbeispiel ein Filter 8, beispielsweise ein Interferenzfilter vor dem Objektiv der Kamera 2 vorgesehen, der zum Hindurchlassen eines bestimmten Wellenlängenbereichs und zum Ausblenden nicht erwünschter Wellenlängen dient.

Die Kamera 2, der Filter 8, der Polarisationsfilter 4 und die Antriebseinheit 5 können in einem Gehäuse angeordnet sein. Dieses Gehäuse kann separat neben der Lichtquelle 1 vorgesehen sein. Bevorzugt ist es jedoch in den Aufbau der Operationsleuchte integriert, z.B. inmitten einer Anordnung von LEDs vorgesehen. Das Gehäuse kann mit einem abnehmbaren Griff versehen sein, der sterilisierbar ist. Auf diese Weise kann der Operateur die Operationsleuchte auf die gewünschte Position einstellen.

Die Kamera ist mit einer Auswerteeinheit 9 verbunden, die beispielsweise als EPLD (Erasable Programmable Logic Device) mit vorgeschalteten 8 Bit AD-Wandler ausgebildet sein kann. Die Auswerteeinheit 9 ist mit einem Monitor verbunden, auf dem das mit der Kamera aufgenommene Bild der Operationsstelle angezeigt wird. Ferner ist die Auswerteeinheit 9 mit einer Steuereinheit 10 verbunden, welche die Antriebe der Polarisationsfilter steuert. Die Auswerteeinheit, die Steuereinheit und die Antriebe bilden ein mechatronisches System. Auf Grundlage der Auswerteergebnisse der Auswerteeinheit steuert die Steuereinheit die mechatronischen Antriebe der Polarisationsfilter.

Im Betrieb wird das unpolarisierte Licht L der Lichtquelle 1 durch den zweiten Polarisationsfilter 6 (teil-) polarisiert. Dieses (teil-)polarisierte Licht LP trifft auf die Operationsstelle 3 auf. Die Operationsstelle 3 ist derart vorbereitet, daß spezifische Strukturen 3a einen Fluorophor, beispielsweise Fluorescein, enthalten. Beispielsweise kann der Fluorophor in das Gewebe appliziert und über drainierende Lymphbahnen zum zugehörigen Lymphknoten transportiert werden. Der Fluorophor wird durch das von der Lichtquelle 1 ausgestrahlte schmalbandige Licht (Anregungslicht) zur Fluoreszenz angeregt und emittiert das (unpolarisierte) Fluoreszenzlicht F, welches üblicherweise eine größere Wellenlänge als das Anregungslicht aufweist. Gleichzeitig wird jedoch das Anregungslicht teilweise an bestimmten Bereichen in der Operationsstelle reflektiert. Dabei kann es sich um bestimmte Gewebebereiche oder aber auch um Flächen von Instrumenten handeln.

Das Fluoreszenzlicht F und das reflektierte Anregungslicht L_{RP} treffen auf den ersten Polarisationsfilter 4. Da das Fluoreszenzlicht unpolarisiert ist, wird es hindurchgelassen. Das reflektierte Anregungslicht L_{RP} ist polarisiert und wird je nach Drehstellung des ersten Polarisationsfilters voll, gar nicht oder teilweise hindurchgelassen. Das Fluoreszenzlicht F und die Reflexionen des Anregungslichts L_{RP} passieren anschließend den Filter 8, durch den Licht mit anderer Wellenlänge als der des Wellenlängenbereichs, den der Filter hindurchläßt und das beispielsweise von zusätzlicher Beleuchtung durch Weißlicht stammt, ausgelöscht wird. Die Kamera 2 erzeugt ein Bild der Operationsstelle 3.

Mit Hilfe der digitalen Bildverarbeitung wird sodann in der Auswerteeinheit 9 in Echtzeit die Summe aller Bildpunkte der CCD-Matrix ausgewertet. Die Auswertung erfolgt mit Hilfe eines Algorithmus, zum Beispiel Bestimmung des Minimums der Bildhelligkeit, Bildung eines Summenintegrals etc.. Gemäß einer Ausführungsform der Auswertung wird die Bildhelligkeit über den betrachteten Bereich integriert.

Durch Verdrehen eines oder beider Polarisationsfilter ändert sich die Bildhelligkeit. Mit Hilfe der mechatronischen Antriebseinheit 5 bzw. 7 wird einer oder werden beide Polarisationsfilter so lange gedreht, bis das Ergebnis der Auswertung eine minimale Bildhelligkeit ist. Dabei ist die Reduzierung der Bildhelligkeit hauptsächlich auf die Auslöschung der Reflexionen des Anregungslichts zurückzuführen.

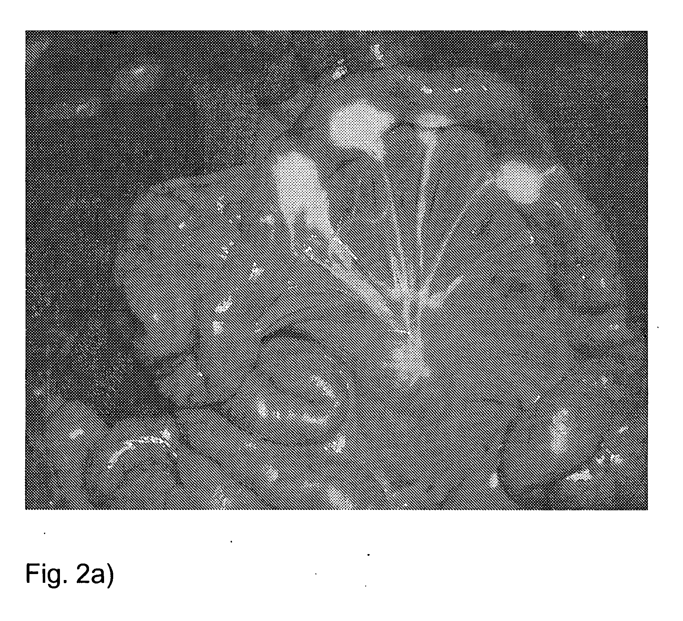

Fig. 2a) zeigt eine Operationsstelle mit fluoreszierenden Lymphbahnen und des zugehörigen Lymphknotens sowie weiteren hellen Bereichen, die das reflektierte Anregungslicht zeigen.

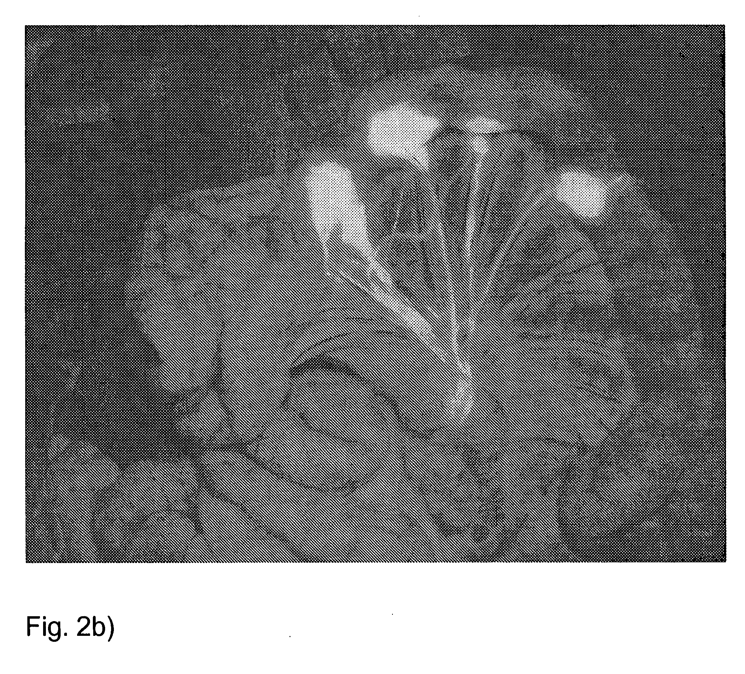

Fig. 2b) zeigt dieselbe Operationsstelle wie Fig. 2a), jedoch mit relativ zueinander im Vergleich zu Fig. 2a) gedrehten Polarisationsfiltern 4, 6. Aufgrund der Drehung der Polarisationsfilter zueinander werden die reflektierten Anteile des Anregungslichts ausgelöscht, während die fluoreszierenden Bereiche deutlich abgrenzbar sind.

Die Einstellung der Polarisationsfilter verläuft automatisch ohne Intervention des Operateurs, so daß dieser sich auf die Operation konzentrieren kann.

Die Aufnahmeparameter der Kamera wie beispielsweise die Blendenöffnung, der Weißabgleich, die Empfindlichkeit etc. sind aufgrund der unterschiedlichen Helligkeiten und unterschiedlichen Spektren bei Beleuchtung mit Weißlicht und bei Fluoreszenzbeleuchtung verschieden. Die Kameraparameter können abgespeichert werden und in Abhängigkeit der verwendeten Beleuchtung abgerufen werden. Dadurch kann die Bildwidergabe optimiert werden.

Abwandlungen des beschriebenen Ausführungsbeispiels sind denkbar. Die Anzahl der Polarisationsfilter ist nicht auf zwei beschränkt. Beispielsweise kann auch nur ein Polarisationsfilter auf der Seite der Kamera vorgesehen sein. Es wird dann mit unpolarisiertem Anregungslicht bestrahlt, was für bestimmte Anwendungen ausreichend sein kann.

Es können mehrere Wellenlängenfilter vorgesehen sein.

Die Kamera kann beispielsweise auch als HDTV-Kamera oder irgendeine andere Kamera ausgebildet sein.

Bei der Auswertung kann gemäß einer weiteren Modifikation anstelle des Integrals der Bildhelligkeit über das gesamte Bild auch die Helligkeitsverteilung im gesamten Bild bestimmt werden.

## Patentansprüche

1. Verfahren zum Ausleuchten einer Operationsstelle mit einer Operationsleuchte mit
einer Lichtquelle (1);
einer Kamera (2), und
wenigstens einem Polarisationsfilter (4), der drehbar ist, wobei wenigstens ein weiterer Polarisationsfilter (6) vorgesehen ist, so dass ein Polarisator und ein Analysator gebildet sind, und wobei weiter eine Steuerung (10) vorgesehen ist, die den Polarisationsfilter (4, 6) in Abhängigkeit von den Ergebnissen einer Auswertung des von der Kamera aufgenommenen Bildes dreht,
**dadurch gekennzeichnet, dass**
die Lichtquelle (1) schmalbandiges Anregungslicht in einem Wellenlängenbereich, der in einem Wellenlängenbereich liegt, in dem ein applizierter Fluorophor empfindlich ist, ausstrahl, wodurch der applizierte Fluorophor zur Fluoreszenz angeregt wird und Fluoreszenzlicht (F)
emittiert, und ein Filter (8) zum Hindurchlassen-von Reflexionen des Anregungslichts und von Fluoreszenzlichts (F) vor dem Lichteintritt in die Kamera (2) verwendet wird,
wobei
die Drehung des Polarisationsfilters (4, 6) in Abhängigkeit von den Ergebnissen der Auswertung des von der Kamera aufgenommenen Bildes erfolgt,
wobei die Drehung automatisch erfolgt, und
wobei der Fluorophor in einem Gewebe enthalten ist, und
die Operationsstelle (3) mit polarisiertem Anregungslicht (L_{Rp}) für Fluoreszenzstrahlung beleuchtet wird und wobei der oder die Polarisationsfilter so gedreht wird bzw. werden, bis die gesamte Helligkeit des betrachteten Bereichs im aufgenommenen Bild minimal ist.

2. Verfahren nach Anspruch 1,
wobei der oder die Polarisationsfilter so lange gedreht wird bzw. werden, bis die Anzahl oder die Fläche von heller beleuchteten Bereichen im aufgenommenen Bild minimal ist.

3. Verfahren nach Anspruch 1 oder 2,
wobei der Filter (8) zum Hindurchlassen eines Wellenlängenbereichs vor dem Objektiv der Kamera verwendet wird.

## Claims

1. Method for illuminating an operation site by means of a surgical lamp with
a light source (1),
a camera (2), and
at least one polarization filter (4) which is rotatable, wherein at least one further polarization filter (6) is provided so that a polarizer and an analyzer are constituted, and wherein, furthermore, a controller (10) rotating the polarization filter (4, 6) depending on the results of an evaluation of the image captured by the camera is provided,
**characterized in that**
the light source (1) emits narrow-band excitation light in a wavelength range being in a wavelength range in which an applied fluorophore is sensitive, whereby the applied fluorophore is excited to fluorescence and it emits fluorescence light (F), and a filter (8) for allowing to pass through reflections of the excitation light and fluorescence light (F) is used in front of the light entrance of the camera (2),
wherein
the rotation of the polarization filter (4, 6) happens depending on the results of the evaluation of the image captured by the camera,
wherein the rotation happens automatically, and
wherein the fluorophore is contained in a tissue, and the operation site (3) is illuminated by polarized excitation light (L_{RP}) for fluorescence radiation, and
wherein the polarization filter(s) is/are rotated such that the entire brightness of the observed region in the captured image is minimum.

2. Method according to claim 1, wherein the polarization filter(s) is/are rotated until the number or the area of brighter illuminated regions in the captured image is minimum.

3. Method according to claim 1 or 2, wherein the filter (8) for allowing to pass a wavelength range is used in front of the lens of the camera.

## Revendications

1. Procédé pour illuminer un poste d'opération comprenant une lampe d'opération avec
une source de lumière (1),
une caméra (2), et
au moins un filtre de polarisation (4), qui peut tourner, au moins un autre filtre de polarisation (6) étant prévu, de sorte qu'un polarisateur et un analyseur sont formés, et également une commande (10) étant prévue, laquelle fait tourner le filtre de polarisation (4, 6) en fonction des résultats d'une analyse de l'image enregistrée par la caméra,
**caractérisé en ce que**
la source de lumière (1) diffuse de la lumière d'excitation à bande étroite dans une plage de longueurs d'onde qui se situe dans une plage de longueurs d'onde dans laquelle un fluorophore appliqué est sensible, de sorte que le fluorophore appliqué est excité pour la fluorescence et émet de la lumière de fluorescence (F), et un filtre (8) est utilisé pour laisser passer des réflexions de la lumière d'excitation et la lumière de fluorescence (F) devant l'entrée de la lumière dans la caméra (2),
dans lequel
la rotation du filtre de polarisation (4, 6) s'effectue en fonction des résultats de l'analyse de l'image enregistrée par la caméra,
la rotation s'effectue automatiquement, et
le fluorophore est contenu dans un tissu, et
le poste d'opération (3) est éclairé avec de la lumière d'excitation (L_{Rp}) polarisée avec du rayonnement de fluorescence et
le ou les filtres de polarisation sont tourné(s) jusqu'à ce que l'ensemble de la luminosité de la zone concernée dans l'image enregistrée soit minimal.

2. Procédé selon la revendication 1, dans lequel le ou les filtres de polarisation sont entouré(s) jusqu'à ce que le nombre ou la surface de zones éclairées de façon plus claire dans l'image enregistrée soit minimal(e).

3. Procédé selon la revendication 1 ou 2, dans lequel le filtre (8) est utilisé pour laisser passer une plage de longueurs d'onde devant l'objectif de la caméra.
